# EUROPEAN PATENT SPECIFICATION

(11) **EP 3 335 044 B1**
(45) Date of publication and mention of the grant of the patent: **19.08.2020**
(21) Application number: 16753876.8
(22) Date of filing: 11.08.2016
(51) Int. Cl.: C07K 7/06, C07K 7/08, G01N 33/53

(54) **ASBESTOS DETECTION**
ASBESTDETEKTION
DÉTECTION DE L'AMIANTE

(30) Priority: 14.08.2015 GB 201514436
(43) Date of publication of application: 20.06.2018
(60) Divisional application: 20182572.6
(73) Proprietor: Centre Scientifique et Technique De la Construction (CSTC), 1000 Brussels (BE); Gesval s.a., 4031 Angleur (BE)
(72) Inventor: BEBRONE, Carine, 4802 Heusy (BE); VANDEGAART, Hélène, 4654 Charneux (BE); NICAISE, Dominique, 1000 Bruxelles (BE)
(74) Representative: Gevers Patents
(86) International application number: PCT/EP2016/069126
(87) International publication number: WO 2017/029190

(56) References cited:
- EP-A1- 1 953 549
- US-A1- 2014 170 773
- TAKENORI ISHIDA ET AL: "Molecular Engineering of a Fluorescent Bioprobe for Sensitive and Selective Detection of Amphibole Asbestos", PLOS ONE, vol. 8, no. 9, 27 September 2013 (2013-09-27), page e76231, XP055316223, DOI: 10.1371/journal.pone.0076231
- KURODA A ET AL: "Detection of chrysotile asbestos by using a chrysotile-binding protein", BIOTECHNOLOGY AND BIOENGINEERING, WILEY & SONS, HOBOKEN, NJ, US, vol. 99, no. 2, 1 February 2008 (2008-02-01), pages 285-289, XP002498959, ISSN: 0006-3592, DOI: 10.1002/BIT.21588 [retrieved on 2007-08-06]

### SEQUENCE LISTING

<110> Symbiose Biomaterials
<120> Asbestos Detection
<130> Arc IP: P0732
<160> 13
<170> PatentIn version 3.5
<210> 1
   <211> 7
   <212> PRT
   <213> bacteriophage M13
<400> 1
   Asp Ala Val Pro Ile Phe Met
   1               5
<210> 2
   <211> 7
   <212> PRT
   <213> bacteriophage M13
<400> 2
   Glu Leu Ala Asn Gln Ser Leu
   1               5
<210> 3
   <211> 7
   <212> PRT
   <213> bacteriophage M13
<400> 3
   Thr Tyr Ala Leu Pro Thr Thr
   1               5
<210> 4
   <211> 7
   <212> PRT
   <213> bacteriophage M13
<400> 4
   Ala Asp Ile Arg His Ile Lys
   1               5
<210> 5
   <211> 7
   <212> PRT
   <213> bacteriophage M13
<400> 5
   Leu Glu Thr Val Val Ser Ser
   1               5
<210> 6
   <211> 12
   <212> PRT
   <213> bacteriophage M13
<400> 6
   Gln Val Asn Gly Leu Gly Glu Arg Ser Gln Gln Met
   1               5                   10
<210> 7
   <211> 7
   <212> PRT
   <213> bacteriophage M13
<400> 7
   His Tyr Met Ser Gly Thr Thr
   1               5
<210> 8
   <211> 7
   <212> PRT
   <213> bacteriophage M13
<400> 8
   Arg Asp His Thr Ala Thr Trp
   1               5
<210> 9
   <211> 7
   <212> PRT
   <213> bacteriophage M13
<400> 9
   Gly His Cys Ser His Pro Thr
   1 5
<210> 10
   <211> 12
   <212> PRT
   <213> BACTERIOPHAGE M13
<400> 10
   Phe Tyr Ser His Ser Ala Asp Ile Arg His Ile Lys
   1 5 10
<210> 11
   <211> 12
   <212> PRT
   <213> bacteriophage M13
<400> 11
   Phe Tyr Ser His Ser Lys Asn His Asp Trp Gly Val
   1 5 10
<210> 12
   <211> 12
   <212> PRT
   <213> bacteriophage M13
<400> 12
   Phe Tyr Ser His Ser Ala Leu Tyr Arg Thr His Thr
   1 5 10
<210> 13
   <211> 9
   <212> PRT
   <213> bacteriophage M13
<400> 13
   Cys Ser Thr Ser Thr Ser Arg Thr Cys
   1 5

## Description

The present invention relates to a biological method for the detection of the presence of asbestos in a sample, more specifically a method making use of bioengineered peptides that specifically bind to selected asbestos fibers ("asbestos-binding peptides") in a sample. In a further aspect, the present invention relates to asbestos-binding peptides as well as to a kit for the detection of the presence of asbestos in a sample.

Asbestos is a general term covering different types of naturally existing fibrous silicate-based minerals. Six different types of asbestos classified in two different mineral groups have been acknowledged so far: the chrysotile type, part of the serpentine mineral group, and the amosite type, the crocidolite type, the tremolite type, the actinolite type and the anthophyllite type, all part of the amphibole mineral group. These different types differ by composition and shape. The chrysotile form accounts for 94% of the worldwide asbestos market.

Asbestos is known for its mechanical strength, chemical and thermal stability, more specifically resistance to fire. Thanks to these characteristics, it has been used in many applications ranging from car brake pads, fire blankets, filters, construction materials, concrete reinforcement, window sills, building panels, construction bricks and blocks, ceiling panels, plaster and plaster boards, roof tiles, roof and wall covers, water and sewage pipes to insulating material. These materials may comprise from about 2 to 20 % wt. of asbestos fibers. Nevertheless, given its hazardous nature, it has been prohibited in many countries. In fact, asbestos fibers are extremely thin (often less than 0.1 µm) and known as a carcinogenic agent, more specifically for their tendency to cause pleural mesothelioma and lung cancer, particularly because of their capacity to easily become air borne and inhaled.

Even in countries where it has been prohibited, many constructions still comprise asbestos-containing materials which can present a risk for residents and workers, more specifically in case of refurbishment of older buildings. In situations where the presence of asbestos is suspected, the construction site is put on standstill until test results from relevant analyses become available and decision is taken on how to proceed further in order to reduce the risk of exposure, particularly worker exposure, to carcinogenic airborne asbestos fibers.

There is thus a need for effective and reliable detection methods that are capable of generating results rapidly to confirm the presence or absence of asbestos.

### Prior Art

Different methods for asbestos detection are known. Laboratory methods include optical and electronic microscopy and X-ray diffraction. More recently, a near-infrared (NIR) detection device (PhazIR™, Polychromix, USA) has been developed for in situ determination of the presence of asbestos. This method nevertheless still requires specifically trained personnel for the performance of the relevant tests. Also, the quantitative determination of asbestos requires additional resources. There is thus an ongoing interest in easy-to-use asbestos detection methods and kits, more specifically such methods and kits that allow for in-situ use with fast results.

With this aim in mind, EP-1953549 proposes contacting an asbestos-binding protein in a solution containing at least 0.1 M sodium chloride with asbestos in a sample and to detect the presence of this protein fused to a reporter protein. In the examples, data is provided for DksA protein from *Escherichia coli* as asbestos-binding protein. The detection method described is a colorimetric detection method making use of a DksA fused to alkaline phosphatase. In an alternative method, the DksA is fused to Green Fluorescent Protein (GFP) allowing the detection of chrysotile type asbestos by fluorescence microscopy. Ishida et al., Selective detection of airborne asbestos fibers using protein-based fluorescent probes. Environ Sci Technol. 2010 Jan 15; 44(2):755-9 then went on to use a bacterial protein called GatZ in a fluorescence based method to selectively determine the presence of amosite and crocidolite.

T Ishida et al, Molecular Engineering of a Fluorescent Bioprobe for Sensitive and Selective Detection of Amphibole Asbestos, Plos One. September 2013, vol 8, Issue 9, e76231, (correlated patent application US2014/0170773) found that GatZ and H-NS protein were not sufficiently specific for amphibole type asbestos as they also bind to wollastonite, an asbestos substitute. The authors speculate that GatZ protein fragments are unlikely to be suitable and propose to look for H-NS protein fragments that specifically bind to amphibole asbestos fibers represented by amosite type asbestos fibers. After evaluating several fragments, the authors found that H-NS₆₀₋₉₀ specifically binds to amosite type asbestos without binding to wollastonite. The fragments, however, showed rather weak affinity to amosite and the authors had to engineer streptavidin-based protein fragment tetramer in order to improve the protein fragment affinity to amosite asbestos. The authors went on to further engineer the peptides but stick to the idea of engineering streptavidin based tetramers showing multiple binding sequences. Also, the authors conclude that the presence of lysine side chains is the most important determinant of the peptide interaction with asbestos.

The proposed detection methods appear to make use of complex engineered molecules to come to a selectivity towards amphibole asbestos.

Efforts have also been directed at identifying small peptides that bind with high affinity to inorganic materials. These specific peptides are called "genetically engineered peptide or polypeptide for inorganics". They are defined as a sequence of amino acids that specifically and selectively binds to an inorganic surface. Whereas the molecular mechanisms of peptide recognition and binding to solid materials are not well understood, some studies showed that the binding is largely governed by electrostatic interactions.

An advantage of inorganic-binding peptides is their high specificity that depends on (i) the chemical composition, (ii) the structuration (powder or flat surface) and the (iii) crystallographic form of the inorganic target substrate. This specificity gives them interesting properties and several applications have already been highlighted (C Tamerler et al, Molecular biomimetics: utilizing nature's molecular ways in practical engineering, Acta biomaterialia, 2007, 3(3) 289-99). Inorganic-binding peptides display useful characteristics and could be used as molecular erectors to direct the assembly of hybrid materials with control of composition and topography. Genetic fusion of these inorganic-binding peptides to functional proteins enables them to be used as linkers for many bio-nanotechnological or microelectronic applications. Inorganic-binding peptides may also be used for self-oriented immobilization of enzymes with conservation of their enzymatic activity. A fivefold repeat of gold-binding-peptide has been fused to the alkaline phosphatase enzyme. The resulting bifunctional enzyme is then self-immobilized, homogenously covering the gold substrate.

### Aims of the Invention

The purpose of the present invention now is to provide an alternative method using asbestos-binding peptides for the detection of asbestos in a sample preferably in the form of a dispersion or suspension in a liquid medium, such as a sample of construction material or of biological material or an air sample.

Another aim of the invention is to provide a simple detection method that allows easy determination in situ of the presence or absence of asbestos.

The method should detect the asbestos type or types most commonly used in construction materials, which is essentially the chrysotile type. Preferably, it should also detect amphibole types of asbestos. The method obviously needs to be selective towards other fibers, such as glass fibers or stone or glass wool fibers.

In addition, the invention seeks to provide novel asbestos-binding peptides specifically and selectively binding to asbestos substrates.

A further purpose of the present invention is to provide a kit for the detection of asbestos fibers in a sample, the kit being easy to use and suitable for on-site use.

Further, the kit should give a result in a short period of time.

### Description of the Invention

Accordingly, the present invention provides a method for the detection of asbestos in a sample, comprising :
(a) contacting a sample with one or more peptides having from 5 to 50 amino acids comprising a motif S/TXS/T, wherein S/T means either serine or threonine amino acid in the sequence and X is an amino acid selected from A, H and R; and
(b) detecting the presence of peptides bound to asbestos fibers.

The peptide may comprise from 5 to 30, more preferably from 7 to 15 amino acids.

It has been found that the method as described above allows for specific binding of the relevant peptides to asbestos fibers. By including an appropriate step to separate bound peptides from unbound peptides, for instance a step aiming at removing unbound peptides, such as a washing step, the peptides bound to asbestos fibers may be retained and may be detected by appropriate detection methods, such as fluorescence techniques, colorimetric techniques and other methods known per se and within the ambit of the knowledge of the skilled person.

In the context of this description, "asbestos-binding peptide" is meant to include such peptides that bind to asbestos fibers with a sufficient affinity to be measurable with known techniques; and with a selectivity such that binding to undesirable species, such as glass fibers, stone or glass wool, is nul or negligible, that is that non-specific binding to undesirable species remains below a level that would be detectable by the detection method used, more particularly below a level detectable by the human eye in fluorescent or colorimetric detection methods.

To that effect, the one or more asbestos-binding peptides may advantageously be coupled to a reporter molecule. Known reporter molecules may advantageously be selected from fluorescent dyes or pigments, such as fluorescein, Cy3, DyLight488, ATTO488, CF488A, DyLight550, CF555, Cy2, rhodamine green, rhodamine, Cy5, Alexa, CF568, CF594 and DY547.

In an alternative, the one or more asbestos-binding peptides may advantageously be fused, possibly in tandem repeats, to a reporter sequence (amino acid sequence) known in the art. Such reporter sequence may advantageously be selected from fluorescent protein, luciferase, alkaline phosphatase, beta galactosidase, beta-lactamase, diaphorase, peroxidase, maltose-binding protein, laccase, lipase, and glucoside hydrolase.

The asbestos-binding peptides may be obtained by techniques known per se. They may for instance be synthesized chemically or produced in organisms, such as bacteria or yeast. The relevant asbestos-binding peptides may be isolated by phage display from appropriate libraries as will be described in more details below. Other techniques known by the skilled person may also be used.

X is selected from A, H and R.

### Preferred peptides are

H Y M S H T T (SEQ ID NO:7),
R D H T A T W (SEQ ID NO:8),
F Y S H S A D I R H I K (SEQ ID NO:10)
F Y S H S K N H D W G V (SEQ ID NO:11),
F Y S H S A L Y R T H T (SEQID NO:12), and
C S T S T S R T C (SEQ ID NO :13).

As is generally known in the art, the asbestos-binding peptides may comprise an amidation at the C-terminal end of the amino acid sequence.

Advantageously, the one or more asbestos-binding peptides are placed in solution, preferably water or a polar organic solvent, most preferably water.

The sample may be a sample of car brake pads, fire blankets, filters, construction materials, concrete reinforcement, window sills, building panels, construction bricks and blocks, ceiling panels, plaster and plaster boards, roof tiles, roof and wall covers, water and sewage pipes or insulating material or another construction material. Preferably, the sample comprises material collected from the surface of the relevant material to be tested or alternatively from bulk construction material. In the alternative, the sample may be an air sample, that is a sample of air taken at a location where air borne asbestos fibers are suspected, for instance close to a site under construction or renovation. According to a different aspect, the sample may be biological material, such as a biopsy or a post mortem body sample. For the purposes of this patent application, the sample is preferably in the form of a dispersion or suspension in a liquid medium.

The method of the invention described here above may further comprise a step for the quantitative analysis of the asbestos fibers detected. Such may consist in known techniques such as image analysis, e.g. analysis of the images from the fluorescence microscopy.

While T Ishida (see prior art listed above) finds the presence of salt required for DkSA to specifically recognize chrysotile type asbestos, the process of the invention does not appear to be linked to the presence of salt. The presence of salt in the sample prepared for testing is nevertheless not necessarily excluded and may be useful in certain applications.

According to another aspect, the present invention also provides novel asbestos-binding peptides having from 5 to 50 amino acids comprising a motif S/T X S/T, wherein S/T means either serine or threonine amino acid in the sequence and X is amino acid selected from A, H and R.

### Preferred peptides are

H Y M S H T T (SEQ ID NO:7),
R D H T A T W (SEQ ID NO:8),
F Y S H S A D I R H I K (SEQ ID NO:10)
F Y S H S K N H D W G V (SEQ ID NO:11),
F Y S H S A L Y R T H T (SEQ ID NO:12), and
C S T S T S R T C (SEQ ID NO :13) .

The peptides may advantageously comprise an amidation at the C-terminal end of the amino acid sequence.

According to yet another aspect, the present invention relates to a novel group of compounds to be used as asbestos-detecting agents in accordance with the invention. Such compounds comprise one or more peptide as defined above coupled to a reporter molecule or fused to a reporter sequence.

Further, according to the invention, the above-defined peptides may be linked in tandem repeats.

Reporter molecules may advantageously be selected from fluorescent dyes or pigments, such as fluorescein, Cy3, DyLight488, Alexa488, ATTO488, CF488A, DyLight550, CF555, Cy2, rhodamine green, rhodamine, Cy5, Alexa546, Alexa555, Alexa568, Alexa594, CF568, CF594 and DY547 .

Reporter sequences may advantageously be selected from fluorescent protein, luciferase, alkaline phosphatase, beta-galactosidase, beta-lactamase, diaphorase, peroxidase, maltose-binding protein, laccase, lipase, and glucoside hydrolase.

According to a preferred embodiment, the one or more asbestos-binding peptide defined above is fused to a reporter sequence or coupled to a reporter molecule by way of a linker. The linker may be selected from Poly-Gly or Gly-rich linkers the length of which may vary, including GGG, GGGS, GGSSG.

According to yet another aspect, the present invention further provides an asbestos detection kit for the detection of asbestos in a sample, comprising an asbestos detection agent as described above, possibly in solution. The relevant detection kit may advantageously further comprise additional solvent and/or the required hardware, such as beakers and pipettes, preferably one-way pipettes, for the performance of the method described above, that is, more specifically the contacting of the asbestos-binding peptide with the sample, and possibly means to separate bound peptides from unbound peptides, for instance for removing unbound peptides, such as a washing solution, hence allowing for detection of the presence of peptides bound to asbestos fibers.

Furthermore, the asbestos detection kit may comprise additional means for the detection of amphibole asbestos fibers.

The detection kit of the present invention is particularly easy to use, even by rather low qualified personnel, and is capable of providing test results in a short period of time. It has been established so far that results may be obtained within less than approx. 1 hour, preferably less than approx. 0.5 hour, more preferably less than 0.25 hour, more specifically in about 10 min.

### Brief Description of the Figures

Fig. 1 shows microscope pictures under visible light and UV light for the fluorescent detection of asbestos fibers versus glass fibers using asbestos-binding peptide SEQ ID NO:9 (not part of the invention) linked to fluorescein dye;
Fig. 2 shows microscope pictures under visible light and UV light for the fluorescent detection of asbestos fibers versus glass fibers using asbestos-binding peptide SEQ ID NO:7 linked to fluorescein dye;
Fig. 3 shows microscope pictures under visible light and UV light for the fluorescent detection of asbestos fibers in asbestos cement roof tiles using asbestos-binding peptide SEQ ID NO:7 and SEQ ID NO:9 (not part of the invention) linked to fluorescein dye; and
Fig. 4 shows pictures of the chromogenic detection of asbestos fibers versus non-sized glass fibers and sized glass fibers using asbestos-binding peptide of SEQ ID NO:9 (not part of the invention) fused to TEM-1-beta-lactamase.

### Example

In the following the peptides defined by SEQ ID NO: 1, 2, 3, 4, 5 and 9 are not part of the invention.

### Selection of asbestos-binding peptides by phage display

Phage display was the approach used to isolate inorganic-binding peptides recognizing asbestos (chrysotile). Three different libraries expressing random peptides were used: Ph.D.™-12, Ph.D.™-7 and Ph.D.™-7C from New England Biolabs Inc. (NEB). These libraries are based on the filamentous M13 bacteriophage expressing a modification on one of the minor coat proteins known as pill. The modified pill protein is expressed at one end of the viral assembly in five copies. The random peptide is fused to the end of each of these pill proteins. The phage display library Ph.D.™-12 is composed of twelve-amino-acid unconstrained random sequences. The phage display library Ph.D.™-7 is composed of seven-amino-acid unconstrained random sequences. The phage display library Ph.D.™-7C contains seven-amino-acid constrained random sequences. "Constrained" refers to the fact that the random peptide sequence is flanked by cysteines. The cysteines form a disulfide bond that forces peptide fusion into a ring-like structure. This limits the conformational freedom available to the peptide and should ensure a more specific fit to the substrate. All of the libraries have complexities approximately of 10⁹ independent clones.

Experiments were performed according to the panning procedure described in the protocols of the Ph.D.™-12, Ph.D.™-7 and Ph.D.™-7C NEB kits. Three different binding conditions were used (the buffer was 50 mM Tris-Cl, pH 7.5 (TB), 50 mM Tris-Cl, pH 7.5 + 150 mM NaCl (TBS) or 50 mM Tris-Cl, pH 7.5+ 150 mM KNO₃ (TBK)). Briefly, 0.1 to 1 mg of asbestos (chrysotile) fibers were washed with the buffer. 100 µL of phage library was incubated with asbestos (chrysotile) fibers in 150 µl of the TB, TBS or TBK buffers supplemented by 0.1 % Tween-20 for 1 h at room temperature. Unbound phages were then separated from chrysotile-binding phages by decantation. The pellet containing chrysotile with bound phages was washed ten times with the respective buffer. Phages which bound to asbestos (chrysotile) were eluted with 100 µl of 0.2 M glycine- HCl (pH 2.2) for 10 min, and finally neutralized with 15 µL of 1 M Tris-HCl (pH 9.1). Phages were then multiplied on *Escherichia coli* ER2738 (NEB) according to the NEB protocol and used in the next panning procedure (briefly, for phage multiplication, 0.2 mL of an overnight *E. coli* ER2738 culture were mixed with eluted phages, transferred to 20 mL of fresh LB medium in a 250 mL flask, and incubated with shaking (160 rpm) at 37 °C for 5 h). Three iterative rounds of panning were performed (with increasing Tween-20 concentrations, 0.1, 0.3 and 0.5 % respectively). A subtractive panning step against the polystyrene plate used for the selection was also performed. Following the last panning, the eluted phages were titrated on agar plates supplemented with IPTG/XGal. Phages from individual plaques were amplified as described above, and their DNAs were isolated and purified.

### Sequencing of amino acids

Sequencing of the M13 phages after 3 rounds of selection was performed with the 96 gill reverse sequencing primer (5'-CCC TCA TAG TTA GCG TAA CG-3'). Sequences were examined with Chromas Lite software (Technelysium Pty Ltd) and Expasy tools Translate program available on-line. Sequence similarity was identified and aligned using the ClustalW program available on-line.

Following this protocol with the above mentioned libraries lead to the following asbestos-binding peptide sequences:
D A V P I F M (SEQ ID NO:1),
E L A N Q S L (SEQ ID NO:2),
T Y A L P T T (SEQ ID NO:3),
A D I R H I K (SEQ ID NO:4),
L E T V V S S (SEQ ID NO:5)
Q V N G L G E R S Q Q M (SEQ ID NO:6),
H Y M S H T T (SEQ ID NO:7),
R D H T A T W (SEQ ID NO:8),
G H C S H P T (SEQ ID NO:9),
F Y S H S A D I R H I K (SEQ ID NO:10)
F Y S H S K N H D W G V (SEQ ID NO:11),
F Y S H S A L Y R T H T (SEQ ID NO:12),
C S T S T S R T C (SEQ ID NO :13).

### Fluorescent asbestos (chrysotile)-binding peptides

The asbestos-binding peptides were synthesized by Eurogentec (Liege, Belgium) either with a fluorescein dye at the N-terminal and amidation at the C-terminal or with a fluorescein dye on an additional C-terminal lysine or cysteine residue and amidation at the C-terminal.

The asbestos-binding peptides were solubilized in water at 10 mg/ml concentration and the solutions were stored at - 20°C.

### Detection of asbestos (chrysotile) using fluorescent asbestos (chrysotile)-binding peptides

Several buffers and MilliQ water were assessed for the fluorescent detection of pure chrysotile fibers (provided by CSTC, Belgium) and for the specificity *vs* glass fibers. The different buffers were 50 mM Tris-CI, pH 7.5 (TB), 50 mM Tris-CI, pH 7.5 + 150 mM NaCl (TBS), 50 mM Tris-CI, pH 7.5+ 150 mM KNO₃ (TBK), TB + 2 % Tween-20, TBS + 2 % Tween-20, TBK + 2 % Tween-20, PBS + 0.3 % Tween-20, 50 mM phosphate buffer pH 8.0 + 2 % Tween-20, 50 mM phosphate buffer + 0.2 M NaCl, 50 mM phosphate buffer + 0.1 M KCI, 50 mM acetate buffer pH 5.0, 50 mM carbonate buffer pH 10.0 or synthetic sea water (30 g NaCl, 0.8 g KCI, 13.5 g MgSO₄.7H₂O, 0.5 g NaHCO₃, 1.3 g CaCl₂).

500 µl of a 40 µg/ml solution of substrate (chrysotile or glass fibers) dispersion in buffer were centrifuged (13000 rpm, 10 minutes, 4°C) and the supernatant was discarded. 1 ml of buffer was then added to the pellet and this dispersion was vortexed 20 seconds. The supernatant was removed after centrifugation (13000 rpm, 10 minutes, 4°C). 1 ml of buffer solution containing fluorescent asbestos (chrysotile)-binding peptide (50 µg/ml) was added and the resulting dispersion was incubated 10 minutes at room temperature protected from light. Then, the solution was centrifuged (13000 rpm, 10 minutes, 4°C) and the supernatant was discarded. The pellet was washed twice in buffer and the supernatant removed by centrifugation. 30 µl of buffer were added to the pellet and this dispersion was dropped and spread onto a glass slide. Fibers are analyzed by optical microscopy with fluorescence.

Confocal imaging was performed using an Olympus BX60 microscope. Digitized images were acquired using an Olympus DP70 digital camera. Fluorescein was visualized by using an excitation wavelength of 488 nm and the emission light was recorded at 535 nm.

In all these conditions (different buffers, with or without salt, with or without Tween-20, different pH values...), asbestos (chrysotile) fibers appeared fluorescent while glass fibers remained non-fluorescent.

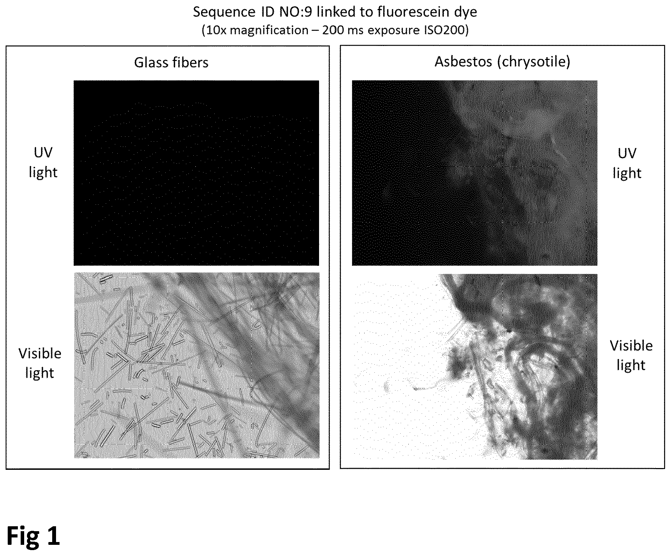

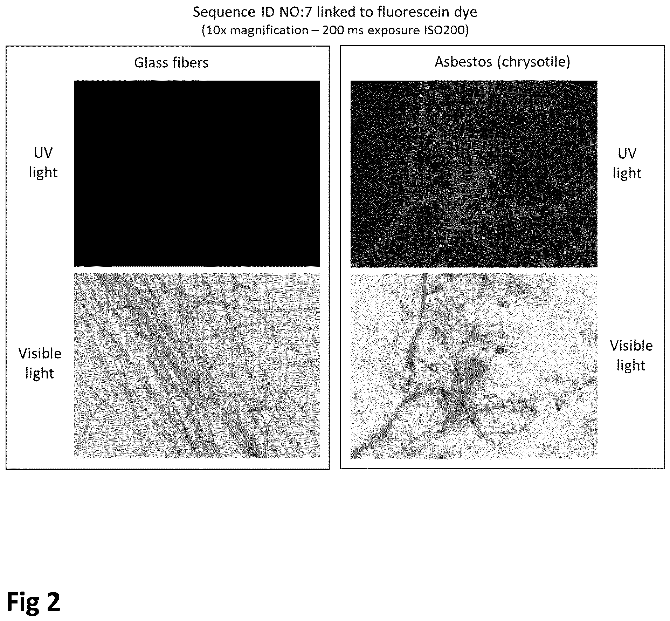

By way of example, Fig. 1 shows microscope pictures under visible light and UV light for the fluorescent detection of chrysotile asbestos fibers versus glass fibers using asbestos-binding peptide SEQ ID NO:9 linked to fluorescein dye; and Fig. 2 shows microscope pictures under visible light and UV light for the fluorescent detection of chrysotile asbestos fibers versus glass fibers using asbestos-binding peptide SEQ ID NO:7 linked to fluorescein dye. As can be seen from the upper left pictures of both figures, the glass fiber sample does not appear to show fluorescence.

### Detection of asbestos (chrysotile) fibers in asbestos-cement roof tiles using fluorescent asbestos (chrysotile)-binding peptides

Asbestos-cement roof tiles contain 15 % of chrysotile (provided by CSTC, Belgium).

Assays on tiles were performed with roughly powdered tiles or directly on the surface.

Detection assay on powder was performed in the following conditions: 0.1 mg/ml of fluorescent asbestos (chrysotile)-binding peptide in PBS buffer + 0.3 % Tween-20, 40 µg/ml of a tile powder dispersion in the same buffer. Incubation time of the fluorescent asbestos-binding peptide with the substrate was 10 minutes. Two washing steps were conducted by centrifugation.

For the detection assay on the surface of the tile, 200 µl of a 0.1 mg/ml solution of fluorescent asbestos (chrysotile)-binding peptide in PBS buffer + 0.3 % Tween-20 were used to inoculate the surface of the tile. Incubation time was 10 minutes. The surface was then largely rinsed with PBS buffer + 0.3 % Tween-20. A last washing step was performed overnight before microscopic observation.

Fluorescence emitted by chrysotile-recognizing peptide was easily distinguishable onto asbestos fibers which could then be identified.

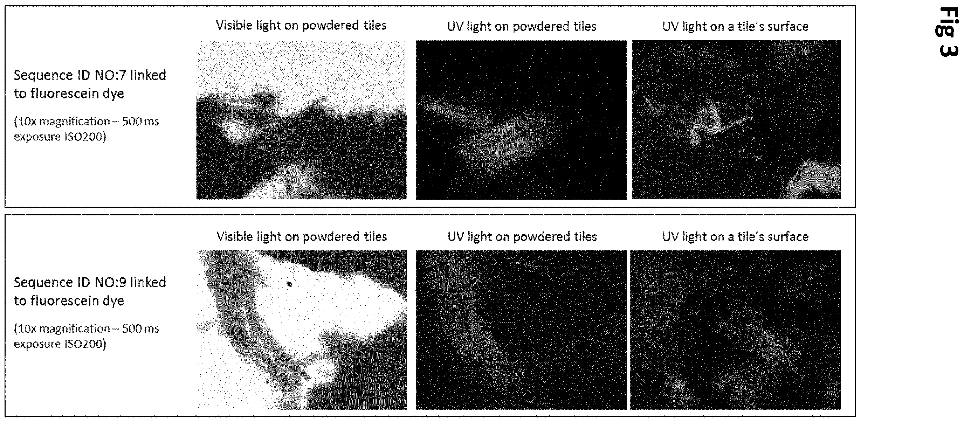

In this connection, Fig. 3 shows microscope pictures under visible light and UV light for the fluorescent detection of chrysotile asbestos fibers in asbestos cement roof tiles, using asbestos-binding peptide SEQ ID NO:7 and asbestos-binding SEQ ID NO:9 linked to fluorescein dye;

### Peptide-TEM protein fusion

Five tandem repeats of the DNA sequence coding for the phage display-selected asbestos binding peptide G H C S H P T (SEQ ID NO:9) were cloned upstream from the gene coding for the TEM-1 β-lactamase (P62593-BLAT_ECOLX). A sequence coding for a GGG linker was introduced between both sequences of interest. *Nco*I and *BamH*I restriction sites were used for cloning in the pET28a surexpression vector (resulting in the pET28a/Peptide-TEM vector).

### Production and purification of Peptide-TEM protein fusion

pET28a/Peptide-TEM expressing *E. coli* was grown overnight at 37°C with shaking in 50 ml LB medium supplemented with 50 µg/ml kanamycin. The bacterial suspension was diluted 100 fold into a total of 1 liter fresh LB medium supplemented with kanamycin (50 µg/ml) and the expression of Peptide-TEM was induced with isopropyl-β-thiogalactopyranoside (final concentration, 1 mM) when the culture reached an A₆₀₀ of 0.6. The induced culture was incubated for 4 h at 37°C with shaking.

The Peptide-TEM protein fusion was purified by ion-exchange chromatography. Briefly, the bacterial suspension was pelleted, resuspended in 30 ml of buffer A (50 mM Tris pH 8.0), disrupted by Emulsiflex high pressure homogenizer and cleared by ultracentrifugation. The supernatant was then dialyzed against buffer A and loaded onto a Q-Sepharose HP column equilibrated with the same buffer. The proteins were eluted with a linear NaCl gradient (0 to 0.5 M). Fractions were analyzed by SDS-PAGE and by their ability to hydrolyze nitrocefin. Those fractions containing the beta-lactamase activity were pooled and dialyzed against buffer A overnight at 4°C. This partially purified enzyme was loaded onto a MonoQ column (GE Healthcare, Diegem, Belgium) equilibrated with the same buffer. The proteins were eluted with a linear gradient of NaCl (0 to 0.5 M). The fractions containing the beta-lactamase activity were pooled and dialyzed against buffer A overnight at 4°C.

### Detection of binding of Peptide-TEM protein fusion to asbestos by coloring

50 µl of Peptide-TEM solution (4 mg/ml) were added to 1 mg of pure asbestos (chrysotile) fibers and the resulting solution was left at room temperature for 10 minutes with gentle shaking for binding. An assay was also performed with asbestos-cement tile powder. In addition, three controls were prepared. One of the controls was obtained without the Peptide-TEM solution, the second with addition of glass fibers (naked or sized) in place of asbestos, and the third without addition of any substrate.

The asbestos (or glass) fibers were precipitated by centrifugation (10000 g, 3 minutes) and after discarding the supernatant, fibers were resuspended in 1 ml of 100 mM phosphate buffer pH 8.0. Then, the fibers were collected by another centrifugation (10000 g, 3 minutes).

The collected fibers were suspended in 100 µl of revelating agent (nitrocefin 200 µM) and left at room temperature for 5 minutes. Note that nitrocefin is generally used as chromogenic substrate of β-lactamases.

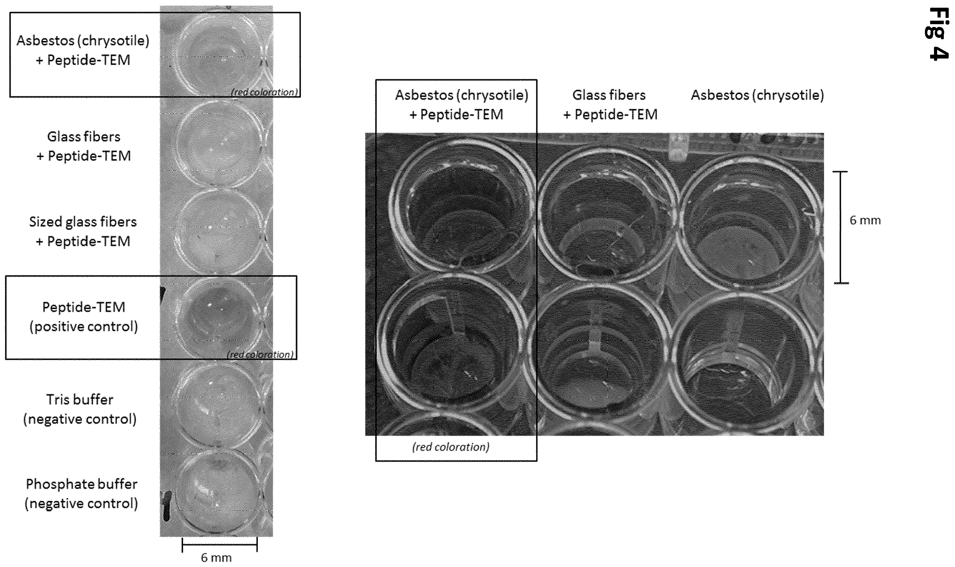

The presence of chrysotile induced the appearance of a red coloration in wells containing pure asbestos fibers or asbestos-cement tile powder while the solutions in wells containing glass fibers (naked or sized) or without substrate remained yellow. See more specifically Fig. 4 which shows pictures of the chromogenic detection of asbestos fibers versus non-sized glass fibers and sized glass fibers using a pentamer of asbestos-binding peptide of SEQ ID NO:9 fused to TEM-1-beta-lactamase.

## Claims

1. A method for the detection of asbestos in a sample, comprising:
(a) contacting a sample with one or more asbestos binding peptides having from 5 to 50 amino acids comprising a motif S/T X S/T, wherein S/T means either serine or threonine amino acid in the sequence and X is an amino acid selected from A, H and R; and
(b) detecting the presence of peptides bound to asbestos fibers.

2. The method according to claim 1, wherein detecting the presence of peptides bound to asbestos fibers comprises fluorescence techniques and/or colorimetric techniques.

3. The method according to any preceding claim, wherein one or more asbestos-binding peptide is fused, possibly in tandem repeats, to a reporter sequence (amino acid sequence), such as a reporter sequence selected from fluorescent protein, luciferase, alkaline phosphatase, beta galactosidase, beta-lactamase, diaphorase and peroxidase, maltose-binding protein, laccase, lipase, and glucoside hydrolase.

4. The method according to any of claims 1 or 2, wherein one or more asbestos-binding peptide is coupled, possibly in tandem repeats, to a reporter molecule selected from fluorescent dyes or pigments, such as fluorescein, Cy3, DyLight488, Alexa488, ATTO488, CF488A, DyLight550, CF555, Cy2, rhodamine green, rhodamine, Cy5, Alexa546, Alexa555, Alexa568, Alexa594, CF568, CF594 and DY547.

5. The method according to any preceding claim, wherein the asbestos-binding peptide is selected from
H Y M S H TT (SEQ ID NO:7),
R D H T A T W (SEQ ID NO:8),
F Y S H S A D I R H I K (SEQ ID NO:10)
F Y S H S K N H D W G V (SEQ ID NO:11),
F Y S H S A L Y R T H T (SEQ ID NO:12), and
C STSTS RTC (SEQ ID NO :13).

6. The method according to any preceding claim, wherein the one or more asbestos-binding peptides are placed in solution, preferably water or a polar organic solvent, most preferably water.

7. The method according to any preceding claim, wherein the sample is a sample of construction material, an air sample or a sample of biological material.

8. The method of any of the preceding claims further comprising an image analysis step for quantitative determination of asbestos fibers.

9. An asbestos-binding peptide having from 5 to 50 amino acids, comprising a motif S/T X S/T, wherein S/T means either serine or threonine amino acid in the sequence and X is an amino acid selected from A, H and R .

10. The asbestos-binding peptide according to claim 9, comprising a C-terminal amidation.

11. The asbestos-binding peptide according to claim 9 or 10, selected from
H Y M S H T T (SEQ ID NO:7),
R D H T A T W (SEQ ID NO:8),
F Y S H S A D I R H I K (SEQ ID NO:10)
F Y S H S K N H D W G V (SEQ ID NO:11),
F Y S H S A L Y R T H T (SEQ ID NO:12), and
C STSTS RTC (SEQ ID NO :13).

12. An asbestos-detection agent comprising one or more peptides according to any of claims 9 to 11, coupled to a reporter molecule or fused to a reporter amino acid sequence, possibly in tandem repeats, possibly by way of a linker selected from Poly-Gly or Gly-rich linkers the length of which may vary, including GGG, GGGS, and GGSSG.

13. A kit for the detection of asbestos in a sample, comprising an asbestos-binding peptide according to any of claims 9 to 11, or an asbestos-detection agent according to claim 12, possibly in solution.

## Patentansprüche

1. Verfahren zum Nachweis von Asbest in einer Probe, umfassend:
(a) Inkontaktbringen einer Probe mit einem oder mehreren asbestbindenden Peptiden mit 5 bis 50 Aminosäuren, die ein Motiv S/T X S/T umfassen, wobei S/T entweder Serin- oder Threonin-Aminosäure in der Sequenz bedeutet, und X eine aus A, H und R ausgewählte Aminosäure ist; und
(b) Nachweisen des Vorhandenseins von an Asbestfasern gebundenen Peptiden.

2. Verfahren nach Anspruch 1, wobei das Nachweisen des Vorhandenseins von an Asbestfasern gebundenen Peptiden Fluoreszenztechniken und/oder kolorimetrische Techniken umfasst.

3. Verfahren nach einem vorstehenden Anspruch, wobei ein oder mehrere asbestbindende Peptide, möglicherweise in Tandemwiederholungen, an eine Reportersequenz (Aminosäuresequenz) fusioniert ist, wie eine Reportersequenz ausgewählt aus fluoreszierendem Protein, Luciferase, alkalischer Phosphatase, Beta-Galactosidase, Beta-Lactamase, Diaphorase und Peroxidase, maltosebindendem Protein, Laccase, Lipase und Glucosidhydrolase.

4. Verfahren nach einem der Ansprüche 1 oder 2, wobei ein oder mehrere asbestbindende Peptide, möglicherweise in Tandemwiederholungen, an ein Reportermolekül gekoppelt ist, ausgewählt aus fluoreszierenden Farbstoffen oder Pigmenten wie Fluorescein, Cy3, DyLight488, Alexa488, ATTO488, CF488A, DyLight550, CF555, Cy2, Rhodamingrün, Rhodamin, Cy5, Alexa546, Alexa555, Alexa568, Alexa594, CF568, CF594 und DY547.

5. Verfahren nach einem vorstehenden Anspruch, wobei das asbestbindende Peptid ausgewählt ist aus
H Y M S H T T (SEQ ID NO: 7),
R D H T A T W (SEQ ID NO: 8),
F Y S H S A D I R H I K (SEQ ID NO: 10),
FY S H S K N H D W G V (SEQ ID NO: 11),
F Y S H S A L Y R T H T (SEQ ID NO: 12) und
C S T S T S R T C (SEQ ID NO: 13).

6. Verfahren nach einem vorstehenden Anspruch, wobei das eine oder die mehreren asbestbindenden Peptide in Lösung gebracht werden, vorzugsweise Wasser oder ein polares organisches Lösungsmittel, am meisten bevorzugt Wasser.

7. Verfahren nach einem vorstehenden Anspruch, wobei die Probe eine Probe aus Baumaterial, eine Luftprobe oder eine Probe aus biologischem Material ist.

8. Verfahren nach einem der vorstehenden Ansprüche, fernerumfassend einen Bildanalyseschritt zur quantitativen Bestimmung von Asbestfasern.

9. Asbestbindendes Peptid mit 5 bis 50 Aminosäuren, die ein Motiv S/T X S/T umfassen, wobei S/T entweder Serin- oder Threonin-Aminosäure in der Sequenz bedeutet, und X eine aus A, H und R ausgewählte Aminosäure ist.

10. Asbestbindendes Peptid nach Anspruch 9, das eine C-terminale Amidierung umfasst.

11. Asbestbindendes Peptid nach Anspruch 9 oder 10, ausgewählt aus
H Y M S H T T (SEQ ID NO: 7),
R D H T A T W (SEQ ID NO: 8),
F Y S H S A D I R H I K (SEQ ID NO: 10),
F Y S H S K N H D W G V (SEQ ID NO: 11),
F Y S H S A L Y R T H T (SEQ ID NO: 12), und
C S T S T S R T C (SEQ ID NO: 13).

12. Asbestnachweismittel, das ein oder mehrere Peptide nach einem der Ansprüche 9 bis 11 umfasst, die, möglicherweise in Tandemwiederholungen, möglicherweise mittels eines Linkers ausgewählt aus Poly-Gly- oder Gly-reichen Linkern, deren Länge variieren kann, einschließlich GGG, GGGS und GGSSG, an ein Reportermolekül gekoppelt oder mit einer Reporteraminosäuresequenz fusioniert sind.

13. Set zum Nachweis von Asbest in einer Probe, das ein asbestbindendes Peptid nach einem der Ansprüche 9 bis 11 oder ein Asbestnachweismittel nach Anspruch 12, möglicherweise in Lösung, umfasst.

## Revendications

1. Procédé permettant de détecter la présence d'amiante dans un échantillon, comprenant les étapes consistant à :
(a) mettre en contact un échantillon avec un ou plusieurs peptides se liant à de l'amiante ayant de 5 à 50 acides aminés comprenant un motif S/T X S/T, où S/T signifie un acide aminé sérine ou thréonine dans la séquence et X représente un acide aminé choisi parmi A, H et R ; et
(b) détecter la présence de peptides liés à des fibres d'amiante.

2. Procédé selon la revendication 1, dans lequel la détection de la présence de peptides liés à des fibres d'amiante comprend des techniques de fluorescence et/ou des techniques colorimétriques.

3. Procédé selon l'une quelconque des revendications précédentes, dans lequel un ou plusieurs peptides se liant à de l'amiante sont fusionnés, éventuellement répétés en tandem, à une séquence rapporteur (séquence d'acides aminés), telle qu'une séquence rapporteur choisie parmi les éléments suivants : une protéine fluorescente, une luciférase, une phosphatase alcaline, une bêta-galactosidase, une bêta-lactamase, une diaphorase et une peroxydase, une protéine se liant au maltose, une laccase, une lipase et une glucoside hydrolase.

4. Procédé selon l'une quelconque des revendications 1 ou 2, dans lequel un ou plusieurs peptides se liant à de l'amiante sont couplés, éventuellement répétés en tandem, à une molécule rapporteur choisie parmi les éléments suivants : colorants ou pigments fluorescents, tels que la fluorescéine, Cy3, DyLight488, Alexa488, ATTO488, CF488A, DyLight550, CF555, Cy2, le vert de rhodamine, la rhodamine, Cy5, Alexa546, Alexa555, Alexa568, Alexa594, CF568, CF594 et DY547.

5. Procédé selon l'une quelconque des revendications précédentes, dans lequel le peptide se liant à de l'amiante est choisi parmi
H Y M S H T T (SEQ ID NO : 7),
R D H T A T W (SEQ ID NO : 8),
F Y S H S A D I R H I K (SEQ ID NO : 10)
F Y S H S K N H D W G V (SEQ ID NO : 11),
F Y S H S A L Y R T H T (SEQ ID NO : 12), et
C S T S T S R T C (SEQ ID NO : 13).

6. Procédé selon l'une quelconque des revendications précédentes, dans lequel le ou les peptides se liant à de l'amiante sont placés en solution, de préférence dans l'eau ou dans un solvant organique polaire, plus préférentiellement dans l'eau.

7. Procédé selon l'une quelconque des revendications précédentes, dans lequel l'échantillon est un échantillon de matériau de construction, un échantillon d'air ou un échantillon de matériau biologique.

8. Procédé selon l'une quelconque des revendications précédentes comprenant en outre une étape d'analyse d'image pour la détermination quantitative des fibres d'amiante.

9. Peptide se liant à de l'amiante ayant de 5 à 50 acides aminés, comprenant un motif S/T X S/T, dans lequel S/T signifie un acide aminé sérine ou thréonine dans la séquence et X représente un acide aminé choisi parmi A, H et R.

10. Peptide se liant à de l'amiante selon la revendication 9, comprenant une amidation C-terminale.

11. Peptide se liant à de l'amiante selon la revendication 9 ou 10, choisi parmi les éléments suivants
H Y M S H T T (SEQ ID NO : 7),
R D H T A T W (SEQID NO : 8),
F Y S H S A D I R H I K (SEQ ID NO : 10)
F Y S H S K N H D W G V (SEQ ID NO : 11),
F Y S H S A L Y R T H T (SEQ ID NO : 12), et
C S T S T S R T C (SEQID NO: 13).

12. Agent de détection d'amiante comprenant un ou plusieurs peptides selon l'une quelconque des revendications 9 à 11, couplé à une molécule rapporteur ou fusionné à une séquence d'acides aminés rapporteur, éventuellement répétés en tandem, éventuellement au moyen d'un lieur choisi parmi les lieurs Poly-Gly ou Gly-riches dont la longueur peut varier, y compris GGG, GGGS et GGSSG.

13. Kit permettant de détecter la présence d'amiante dans un échantillon, comprenant un peptide se liant à de l'amiante selon l'une quelconque des revendications 9 à 11, ou un agent de détection d'amiante selon la revendication 12, éventuellement en solution.
